# EUROPÄISCHE PATENTSCHRIFT

(11) **EP 2 094 211 B1**
(45) Veröffentlichungstag und Bekanntmachung des Hinweises auf die Patenterteilung: **04.01.2012**
(21) Anmeldenummer: 07816234.4
(22) Anmeldetag: 09.11.2007
(51) Int. Cl.: A61F 13/02, A61F 13/00

(54) **VORRICHTUNG ZUR WUNDBEHANDLUNG**
DEVICE FOR TREATING WOUNDS
DISPOSITIF DE TRAITEMENT DE PLAIE

(30) Priorität: 30.11.2006 CH 19432006
(43) Veröffentlichungstag der Anmeldung: 02.09.2009
(73) Patentinhaber: Medela Holding AG, 6340 Baar (CH)
(72) Erfinder: AICHER, Martin, 8049 Zürich (CH)
(74) Vertreter: Clerc, Natalia
(86) Internationale Anmeldenummer: PCT/CH2007/000551
(87) Internationale Veröffentlichungsnummer: WO 2008/064502

(56) Entgegenhaltungen:
- WO-A-00/78212
- WO-A-2007/075379
- US-A- 5 636 643
- US-A1- 2006 235 347

## Beschreibung

### Technisches Gebiet

Die Erfindung betrifft eine Vorrichtung zur Wundbehandlung gemäss Oberbegriff des Patentanspruchs 1.

### Stand der Technik

Offene Wunden, welche zu gross oder zu stark entzündet sind, um selbständig zu heilen, waren lange Zeit ein schwieriges Gebiet der Medizin. Es hat sich gezeigt, dass Wundbehandlung unter Verwendung von Unterdruck die Heilung der Wunde stimuliert, unterstützt bzw. beschleunigt. Diese Art der Behandlung ist unter dem Begriff Wunddrainage bekannt.

US 5 636 643 offenbart beispielsweise eine Wunddrainagevorrichtung mit einer flüssigkeits- und gasdichten steifen Haube, welche über eine Wunde gelegt und ausserhalb der Wundränder auf die gesunde Haut befestigt wird. Unterhalb der Haube ist ein Schaumstoff auf bzw. in die Wunde gelegt. In der Haube wird von aussen mittels einer Vakuumpumpe ein Unterdruck erzeugt, um die Wundheilung zu beschleunigen.

Auch WO 03/018098 beschreibt eine Vorrichtung zur Wundbehandlung mit einer Haube und einem porösen Kissen, welches unter der Haube auf die Wunde gelegt ist. Hier wird vorgeschlagen, den Unterdruck automatisch zu oszillieren, um die Wundheilung zu stimulieren.

WO 2006/056408 schlägt vor, in die Haube Zufuhrvorrichtungen für Behandlungsmedien vorzusehen. Diese Behandlungsmedien werden zusammen mit dem Wundsekret durch eine Abfuhrvorrichtung abgeführt.

WO 2006/048246 offenbart einen Mehrkomponentenverband zur Wundbehandlung mittels Unterdruck. Dieser Verband weist superabsorbierende Polymere auf, wobei die absorbierten Wundsekrete an Polymere gebunden im Wundraum verbleiben.

In US 2006/0155260 wird die Wunde mit einer Flüssigkeit gereinigt, wobei ein geschlossener Kreislauf verwendet wird.

WO 2004/071279 beschreibt eine Vorrichtung zur Wundbehandlung, welche Sensoren aufweist, um die Wundheilung zu überwachen.

WO 2006/081221 schlägt ferner vor, Wunden phototherapeutisch zu behandeln.

WO 00/78212 offenbart eine Vorrichtung zum Fördern der Blutdurchströmung von Hautlappen, zum Beispiel bei kosmetischer Chirurgie.

US 2006/235347 zeigt einen Ring, welcher um eine Wunde gelegt wird.

### Darstellung der Erfindung

Es ist deshalb eine Aufgabe der Erfindung, eine Vorrichtung zur Wundbehandlung zu schaffen, welche für unterschiedliche Wundgrössen einsetzbar ist.

Diese Aufgabe löst eine Vorrichtung zur Wundbehandlung mit den Merkmalen des Patentanspruchs 1.

Die erfindungsgemässe Vorrichtung zur Wundbehandlung eines Patienten, vorzugsweise mittels Unterdruck, weist eine Abdeckung zur Bildung einer Unterdruckkammer über der Wunde auf, wobei die Abdeckung auf der die Wunde umgebenden Haut befestigbar ist. Die Form der Abdeckung ist nach der Aufbringung auf die Wunde und ihrer Befestigung auf der Haut von einer Basisform in eine vordefinierte Gebrauchsform veränder bar.

Diese Wundabdeckung lässt sich auf einfache Weise spezifisch auf die entsprechende Wunde anpassen. Sie verhindert einen mechanischen Druck auf das Wundbett und ermöglicht trotzdem eine Wundkontraktion. Sie ist in ihrer Anwendung zudem relativ einfach, so dass die Anbringung auf die Wunde und Entfernung von der Wunde wenig Zeit benötigt.

Vorzugsweise ist die Abdeckung selbsttätig von der Basisform in die Gebrauchsform veränderbar. Dies hat den Vorteil, dass eine Berührung der Wunde durch unsachgemässe Verwendung der Abdeckung weitgehend vermieden werden kann.

Vorzugsweise besteht die Abdeckung mindestens teilweise, vorzugsweise vollständig, aus einem Material mit Formgedächtnis. Vorzugsweise weist die Abdeckung bei einer Temperatur, welche annähernd einer Körpertemperatur des Patienten entspricht, die Gebrauchsform auf. Die Basisform ist vorzugsweise bei Zimmertemperatur (d.h. ca. 20°C) vorhanden. Es lassen sich alle geeigneten Materialien mit Formgedächtnis verwenden, insbesondere Polymere oder Metalle.

Die Abdeckung kann jedoch auch zusätzlich oder alternativ mit mindestens einem mechanischen Element versehen sein, mittels welchem ihre Form veränderbar ist. Diese kann beispielsweise eine Rückstellfeder sein.

In einer Ausführungsform weist die Abdeckung ein Grundelement ohne Formgedächtnis und mindestens ein auf oder in diesem Grundelement angeordnetes Teilelement mit Formgedächtnis auf, wobei das mindestens eine Teilelement sich derart über das Grundelement erstreckt, dass es bei Veränderung seiner Form das Grundelement zu einer vorgegebenen Formveränderung zwingt.

In einer weiteren Ausführungsform weist die Abdeckung ein zu einem Ring biegbares Profilelement und eine über den Ring spannbare Abdeckfolie auf. Vorzugsweise kann das Profilelement seine Profilhöhe mechanisch, z.B. über eine Feder, über Formgedächtnis, über Aufpumpen oder dank seines Temperaturausdehnungskoeffizienten kontrolliert verändern.

In der Abdeckung können eines oder mehrere der folgenden Mittel angeordnet sein: ein Ultraschallsender, eine Lichtquelle, ein Temperatursensor, ein Sensor zur Messung der Feuchtigkeit, ein Sensor zur Messung der Durchblutung, ein Sensor zur Messung der bakteriellen Besiedelung, eine Heizung.

Die erfindungsgemässe Vorrichtung wird vorzugsweise bei der Wunddrainage mit Unterdruck verwendet. Sie kann jedoch auch für andere Wundbehandlungen verwendet werden. Zudem wird unter Wunde hier nicht nur eine offene Hautstelle sondern auch Hautanomalien oder andere Gewebedefekte verstanden.

Das erfindungsgemässe System zur Wundbehandlung weist eine Vorrichtung zur Wundbehandlung der oben genannten Art sowie eine damit verbindbare Vakuumpumpe auf, zur Erzeugung eines Unterdrucks in einer durch die Vorrichtung zur Wundbehandlung erzeugten Kavität.

Im erfindungsgemässen Verfahren zur Wundbehandlung wird eine Abdeckung auf der Wunde befestigt, wobei die Abdeckung bei der Befestigung eine Basisform aufweist, und wobei nach der Befestigung der Abdeckung diese in eine Gebrauchsform verändert wird.

Weitere vorteilhafte Ausführungsformen und Varianten des Verfahrens gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: einen Querschnitt durch eine erfindungsgemässe Abdeckung in einer Basisform vor ihrer Verwendung gemäss einer ersten Ausführungsform;
- Figur 2a: einen Querschnitt durch die Abdeckung gemäss Figur 1 in einer Basisform;
- Figur 2b: eine perspektivische Darstellung der Abdeckung gemäs Figur 2a;
- Figur 3a: einen Querschnitt durch eine Wunde und umliegendes Gewebe;
- Figur 3b: eine perspektivische Darstellung der Wunde gemäss Figur 3a;
- Figur 4a: einen Querschnitt durch die Wunde gemäss Figur 3a mit darüber angeordneter Abdeckung gemäss Figur 2a;
- Figur 4b: die Wunde und Abdeckung gemäss Figur 4a in perspektivischer Darstellung;
- Figur 5a: einen Querschnitt durch die Wunde und der darüber befestigten Drainagevorrichtung in einer Basisform;
- Figur 5b ,: die Wunde und die Vorrichtung gemäss Figur 5a in perspektivischer Darstellung;
- Figur 6: einen Querschnitt durch die Wunde und die Vorrichtung gemäss Figur 5a in einer Gebrauchsform;
- Figur 7a: eine graphische Darstellung des angelegten Vakuums in Funktion der Zeit;
- Figur 7b: eine graphische Darstellung einer Hydrogel-Verabreichung in Funktion der Zeit;
- Figur 7c: eine graphische Darstellung einer Spülung in Funktion der Zeit;
- Figur 8a: einen Querschnitt durch die Wunde und Drainagevorrichtung einer Hydrogel-Verabreichung in einem ersten Zustand und
- Figur 8b: in einem zweiten Zustand;
- Figur 9: einen Querschnitt durch die erfindungsgemässe Drainagevorrichtung in einer zweiten Ausführungsform;
- Figur 10: einen Querschnitt durch die erfindungsgemässe Drainagevorrichtung in einer dritten Ausführungsform;
- Figur 11 a: einen Querschnitt durch die erfindungsgemässe Drainagevorrichtung in einer vierten Ausführungsform;
- Figur 11b: eine graphische Darstellung der Messung der Durchblutung;
- Figur 12: einen Querschnitt durch die erfindungsgemässe Drainagevorrichtung in einer fünften Ausführungsform;
- Figur 13a: eine perspektivische Darstellung der erfindungsgemässen Drainagevorrichtung in einer sechsten Ausführungsform in einer Basisform und
- Figur 13b: in einer Gebrauchsform;
- Figur 13c: einen Querschnitt durch die Vorrichtung gemäss Figur 13b;
- Figur 14: eine Ansicht einer ersten Variante eines erfindungsgemässen Teilelements;
- Figur 15: eine Ansicht einer zweiten Variante eines erfindungsgemässen Teilelements;
- Figur 16a: eine perspektivische Darstellung eines Profilelements einer Drainagevorrichtung gemäss einer siebten Ausführungsform;
- Figur 16b: das Profilelement gemäss Figur 16a in einer Anordnung um eine Wunde und
- Figur 16c: einen Querschnitt durch die Drainagevorrichtung gemäss der siebten Ausführungsform.

### Wege zur Ausführung der Erfindung

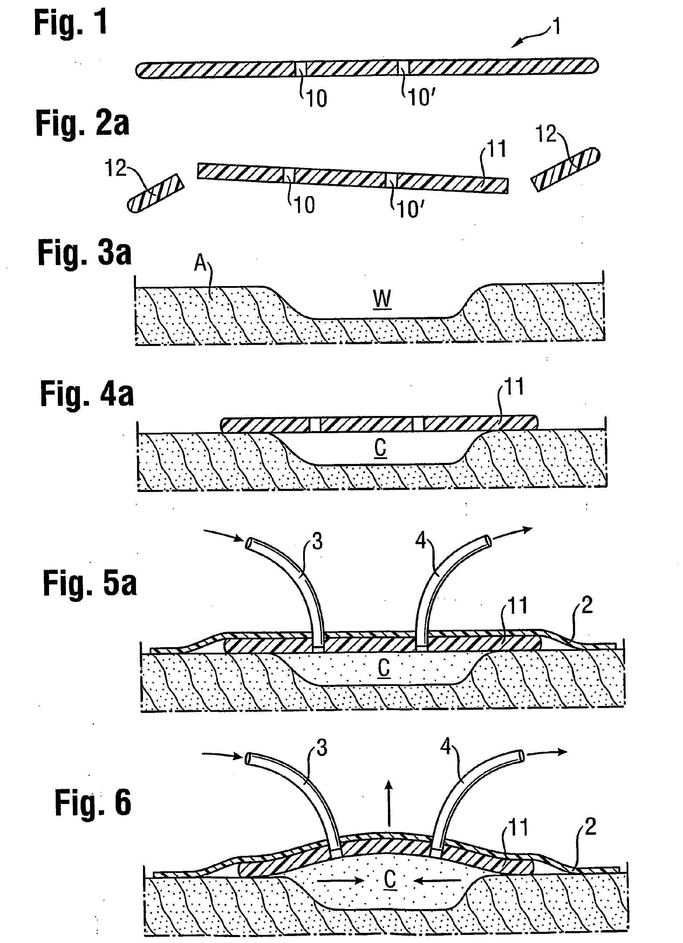

In den Figuren 1 bis 6 ist eine erste bevorzugte Ausführungsform der erfindungsgemässen Vorrichtung zur Wundbehandlung, insbesondere eine Wunddrainagevorrichtung, dargestellt. Ausgangsmaterial ist eine Abdeckplatte 1 gemäss Figur 1, welche vorzugsweise plan, insbesondere planparallel, ausgebildet ist. Diese Platte 1 weist mindestens zwei, eventuell auch drei, vier oder mehr Durchgangsöffnungen 10, 10' auf, welche vorzugsweise senkrecht zur Platte 1 verlaufen. Sie können diese jedoch auch in einem nicht rechtwinkligen Winkel durchdringen.

Die Platte 1 weist ein Formgedächtnis auf, welches vorzugsweise thermisch aktivierbar ist. Vorzugsweise wird eine vordefinierte bzw. vorprogrammierte Form bei menschlicher Körpertemperatur eingenommen.

Vorzugsweise ist das Material ein SMP, d.h. ein Shape Memory Polymer, beispielsweise ein Block-Copolymer, zum Beispiel ein Wachs-Elastomer-Komposit. Vorzugsweise ist die Platte starr oder zumindest selbsttragend ausgebildet, so dass sie sich in ihrer Form nur unter äusserer Krafteinwirkung verändert und insbesondere ihre Basisform wie auch die nachfolgend beschriebene Gebrauchsform auch ohne ganzflächige Unterstützung beibehält. Insbesondere die Gebrauchsform sollte auch bei einem Druckunterschied auf ihren zwei gegenüberliegenden Seiten beibehalten werden. Die Platte 1 weist vorzugsweise eine Dicke von 1 bis 6 mm auf. In einer weiteren Ausführungsform kann das Formgedächtnispolymer durch Magnetismus oder Licht stimuliert werden. Dies gilt auch für andere Ausführungsfbrmen der Erfindung, insbesondere für die weiter unten beschriebenen Ausführungsformen.

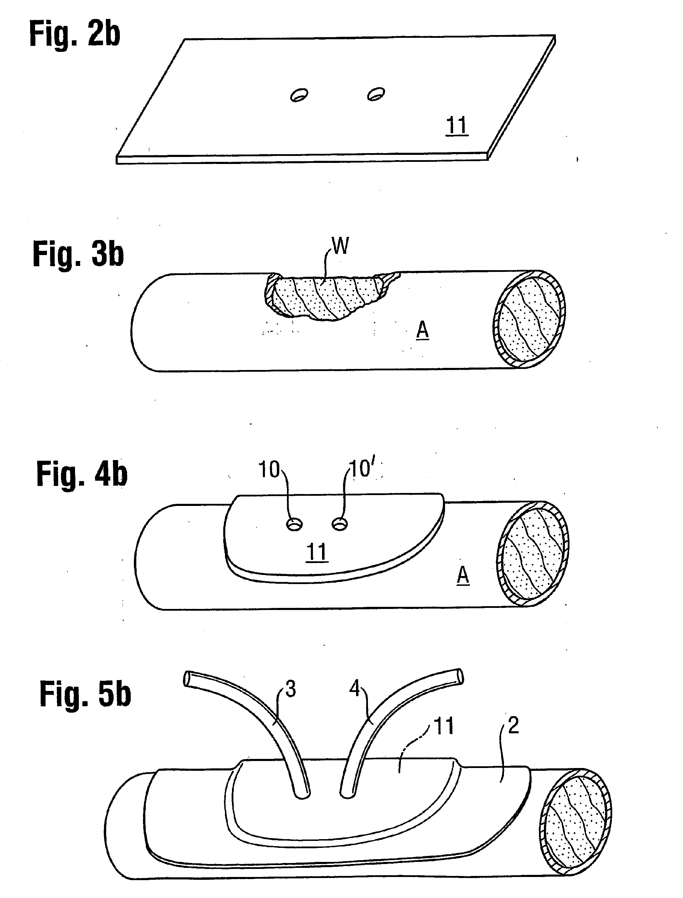

Diese Abdeckplatte 1 wird nun bei Gebrauch auf die Grösse der Wunde angepasst. Eine entsprechende Wunde W ist in den Figuren 3a und 3b dargestellt. Gemäss den Figuren 2a und 2b wird nun die Platte 1 entsprechend zugeschnitten, wobei einfach an zwei gegenüberliegenden Kanten eine gewünschte Kantenbreite 12 abgeschnitten oder abgebrochen wird. Übrig bleibt ein Abdeckplattenzuschnitt 11 mit den zwei Durchgangsöffnungen 10, 10'. Dieser Zuschnitt 11 wird nun gemäss den Figuren 4a und 4b über die Wunde W gelegt, so dass eine Kavität C entsteht. Der Zuschnitt 11 wird gemäss den Figuren 5a und 5b mittels einer Haftfolie 2 oder einem anderen geeigneten Fixierungselement in seiner Lage fixiert. Hierfür wird die Haftfolie 2 an ihren Rändern auf gesunde Haut bzw. Gewebe A, welche die Wunde W umgibt, geklebt. Die Folie 2 kann auch auf dem Zuschnitt 11 kleben. Sie kann jedoch auch einfach darüber gespannt sein ohne mit der Folie verbunden zu werden.

Des weiteren wird in die erste Durchgangsöffnung 10 ein Zuführschlauch 3 und in die zweite Durchgangsöffnung 10' ein Abführschlauch 4 gesteckt bzw. durchgeführt. Die Fixierung der Schläuche 3, 4 kann ebenfalls mittels der Folie 2 erfolgen und/oder sie können mit Fixierungsringen, Klemmen, separaten Klebemitteln oder anderen geeigneten Mitteln in ihrer Lage fixiert werden.

Wie in den Figuren 5a und 5b erkennbar ist, weist der Zuschnitt 11 bei seiner Aufbringung nach wie vor eine Basisform, hier als planparallele Platte, auf. Die Basisform könnte auch eine andere Form sein, sie kann beispielsweise eine Wölbung nach innen oder aussen in Bezug auf die Kavität C aufweisen, sie kann auch gewellt oder pyramidenförmig sein.

Wie nun in Figur 6 erkennbar ist, nimmt der Zuschnitt 11, sobald er die Temperatur des umliegenden Gewebes A bzw. der Wunde W angenommen hat, eine Gebrauchsform ein. Vorzugsweise ist diese Gebrauchsform hauben- oder kalottenförmig, wobei sie sich nach aussen, von der Wunde wegneigt, wie dies der vertikale Pfeil zeigt. Die Höhe der Kavität C und somit der Abstand des Zuschnitts 11 von der Wundoberfläche werden dadurch vergrössert. Mechanischer Druck auf das Wundbett und ein Anwachsen der Abdeckung werden verhindert. Wesentlich ist zudem, dass durch die Formveränderung eine Wundkontraktion stattfindet, da die Wundränder nach innen gezogen werden, wie dies durch die horizontalen Pfeile dargestellt ist und dank dem auch in der Gebrauchslage starren Zuschnitt 11 während der gesamten Drainage bzw. Wundbehandlung ein steter Zug bzw. Druck nach innen auf die Wundränder ausgeübt wird.

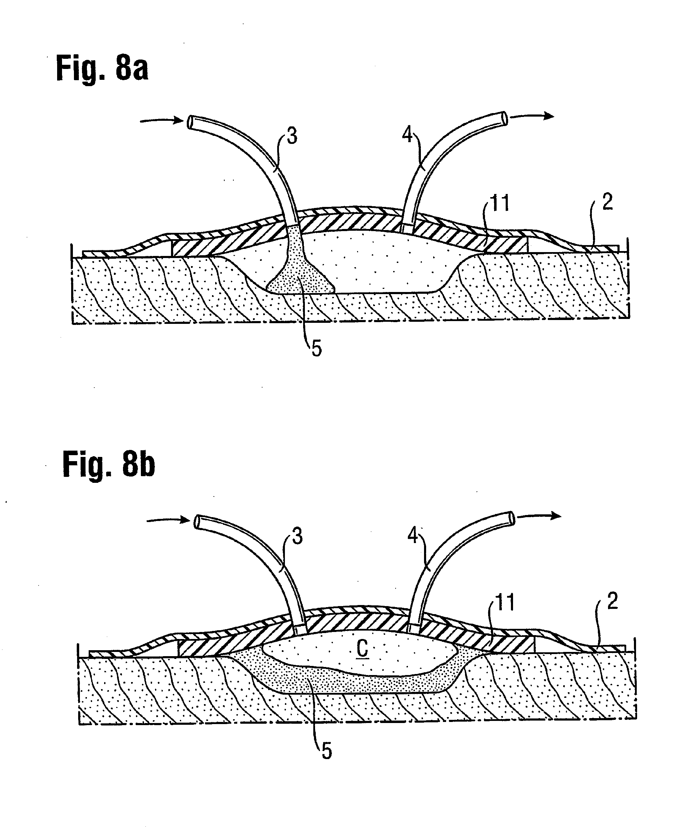

Falls nicht schon begonnen, kann nun die Wunddrainge gestartet werden, d.h. ein geeigneter Unterdruck kann über den Abführschlauch 4 angelegt werden. Wundsekret kann ebenfalls über den Abführschlauch 4 abgeführt werden. Des weiteren lassen sich Mittel zur Beschleunigung der Heilung bzw. zur Desinfektion der Wunde über den Zuführschlauch 3 zuleiten, wie beispielsweise ein Hydrogel 5, wie dies in den Figuren 8a und 8b dargestellt ist. Die Wunde kann auch gespült werden, beispielsweise mit einer Ringer-Lösung.

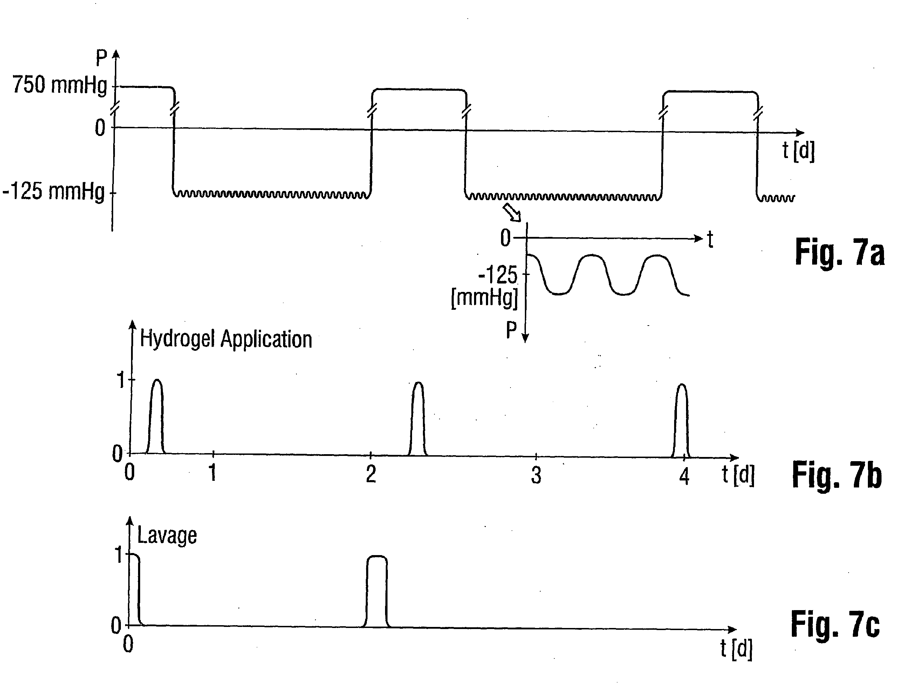

In den Figuren 7a bis 7c ist ein möglicher Zusammenhang zwischen einer Vakuumbeaufschlagung der Kavität C (Figur 7a), einer Hydrogel-Verabreichung (Figur 7b) und einer Spülung der Kavität C (Figur 7c) dargestellt. Die drei Zeitachsen t sind identisch und ein Inkrement bezeichnet jeweils 1 Tag d. Wie in Figur 7a erkennbar ist, wird über längere Zeit ein pulsierendes sinusförmiges Vakuum angelegt. Gemäss Figur ist es circa 125 mmHg, vorzugsweise liegt es zwischen 80 bis 140 mmHg. Die Hydrogel-Verabreichung erfolgt über einen wesentlich geringeren Zeitraum als einen Tag, vorzugsweise während 1 bis 3 Minuten innerhalb von 1 bis 2 Tagen bis zur Benetzung der Wunde. Dasselbe gilt für die Spülung, wobei diese vorzugsweise zeitlich vor der Benetzung des Wundbetts mit Hydrogel erfolgt und die Verabreichung erst 0.5 bis 2 Stunden nach der Spülung gestartet wird. Eine Oxygenierung kann zeitlich parallel zur Unterdruckapplikation oder als eigene Phase zwischen den Applikationen erfolgen.

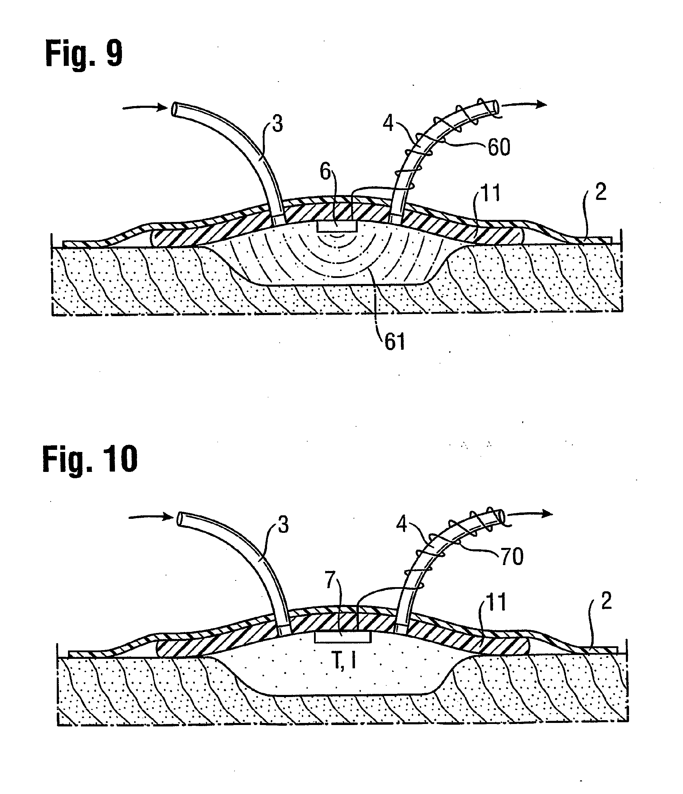

Des weiteren kann die Wunddrainagevorrichtung bzw. die hierfür verwendete Abdeckung mit zusätzlichen Funktionsmitteln versehen werden. So kann, wie dies in Figur 9 dargestellt ist, ein Ultraschallsender 6 mit dem Zuschnitt 11 verbunden sein, welcher Ultraschallwellen 61 in die Kavität C aussendet. Dieser Sender 6 kann bereits herstellerseitig angebracht und als Bestandteil der Abdeckplatte 1 geliefert werden. Er kann jedoch auch nachträglich befestigt werden. Die Abdeckplatte 1 kann hierfür eine entsprechende Ausnehmung oder Einbuchtung aufweisen. Die Befestigung kann mittels Kleben, Klammern, Schweissen, Eingiessen oder anderen geeigneten Mitteln erfolgen. Vorzugsweise wird der Ultraschallsender 6 von demselben Gerät, welches auch die Vakuumquelle beinhaltet, betrieben. In diesem Fall ist es vorteilhaft, wenn die Leitung 60 für den Ultraschallsender 6 entlang des Abführschlauches 4 geführt ist.

Anstelle des Ultraschallsenders 6 kann auch eine Photoquelle vorhanden sein, welche Licht in die Kavität C aussendet. Die Darstellung entspricht derjenigen der Figur 9 und obige Beschreibung gilt auch für die Photoquelle. Ultraschallsender und Photoquelle lassen sich auch gemeinsam in der Wunddrainagevorrichtung verwenden. Beide stimulieren die Wundheilung auf ihre Weise. Sie können gemeinsam oder anstelle der Vakuumbeaufschlagung eingesetzt werden.

Des weiteren bzw. anstelle dieses Senders bzw. dieser Quelle können auch Mittel zur Überwachung der Wundheilung mit dem Zuschnitt 11 verbunden sein. Auch hier gilt obig Gesagtes bezüglich dem Zeitpunkt und der Art der Befestigung. Ein derartiges Mittel ist beispielsweise ein Temperatursensor und/oder ein Hydrosensor 7, welcher die Feuchtigkeit in der Wunde misst. Dies ist in Figur 10 dargestellt. Auch hier wird die Signalleitung 70 vorzugsweise entlang des Abführschlauchs 4 geführt und die Datenauswertung bzw. deren Verwertung für die weitere Behandlung im Gerät der Vakuumpumpe durchgeführt.

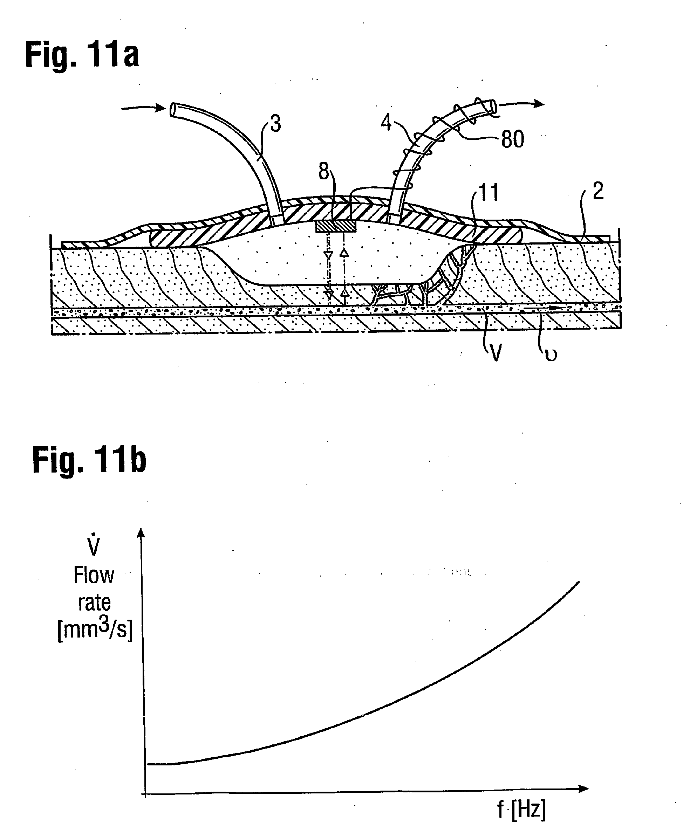

Ein weiteres Überwachungsmittel ist ein Durchflusssensor 8 gemäss Figur 11a, welcher die Durchblutung des unter der Wunde liegenden Gewebes A qualitativ misst und die Daten über die Signalleitung 80 weiterleitet. Figur 11b zeigt ein Beispiel einer Durchflussrate, wie sie vom oben genannten Sensor detektiert werden kann.

Es lassen sich jedoch auch noch andere Überwachungssensoren einsetzen, beispielsweise ein Sensor zur Überwachung der bakteriellen Besiedelung.

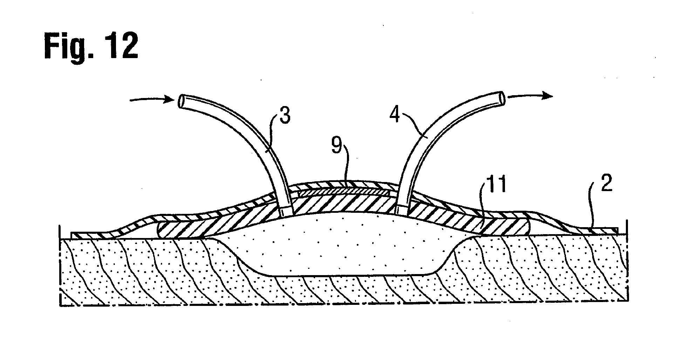

Des weiteren lässt sich die Vorrichtung zusätzlich oder alternativ mit einem Heizelement 9 versehen, um die gewünschte Temperatur aufrechtzuerhalten. Diese Temperatur kann die optimale Temperatur zur Erhaltung der gewünschten Gedächtnisform des Zuschnitts 11 sein. Sie kann aber auch die für die Wundheilung optimale Temperatur sein. Dies ist in Figur 12 dargestellt. Als Heizelement 9 eignet sich insbesondere ein elektrisches Widerstandsheizelement bekannter Art, vorzugsweise eine flächige Ausführungsform. Es kann jedoch auch ein über eine chemische Reaktion aktivierbares Heizelement oder ein anderes geeignetes Heizelement verwendet werden. Hier ist das Heizelement 9 ausserhalb der Kavität C auf der dieser gegenüberliegenden Seite des Zuschnitts 11 angeordnet, wobei es sich unterhalb der Haftfolie 2 befindet. Es kann aber auch in der Kavität C oder über der Haftfolie 2 angeordnet sein. Auch die übrigen, oben beschriebenen Sensoren und Quellen lassen sich, falls hierzu geeignet, ausserhalb der Kavität C anordnen.

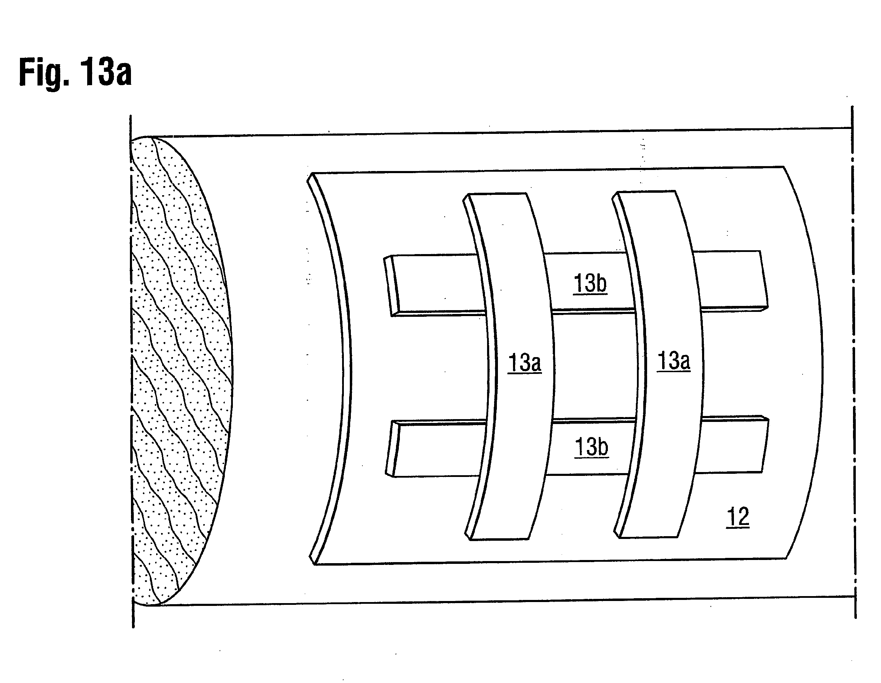

In den Figuren 13a bis 13c ist eine weitere Ausführungsform der erfindungsgemässen Drainagevorrichtung bzw. deren Abdeckung dargestellt. Die Abdeckung weist hier ein plattenförmiges Grundelement 12 auf, deren Grössen sich ebenso wie der Zuschnitt gemäss den obigen Beispielen frei wählen bzw. an die Wunde anpassen lässt. Auf dem Grundelement 12 sind nun Teilelemente in Form von Längs- und Querstreifen 13a, 13b angeordnet und mit diesem mindestens abschnittsweise fest verbunden. Längs-und Querstreifen 13a, 13b verlaufen annähernd senkrecht zueinander. Das Grundelement 12 weist kein Formgedächtnis auf, jedoch diese Streifen 13a, 13b schon. Vorzugsweise ist das Grundelement aus einer fluiddichten Polymerfolie gefertigt und die Streifen 13a, 13b wiederum aus einem Shape Memory Polymer oder einem Shape Memory Metall.

Vorzugsweise wirken die Streifen 13a, 13b als Kontraktionsbänder, wenn sie die entsprechende Temperatur, welche das Formgedächtnis aktiviert, erreicht haben. Diese Temperatur ist auch hier vorzugsweise die menschliche Körpertemperatur, d.h. ca. 37°C. Die Streifen 13a, 13b werden üblicherweise auf die Grösse der Wunde angepasst. Grundelement 12 und Bänder 13a, 13b können als Einheit geliefert und gemeinsam zugeschnitten werden. Vorzugsweise sind sie jedoch Einzelteile, welche zuerst der entsprechenden Wunde angepasst und dann zusammengefügt werden.

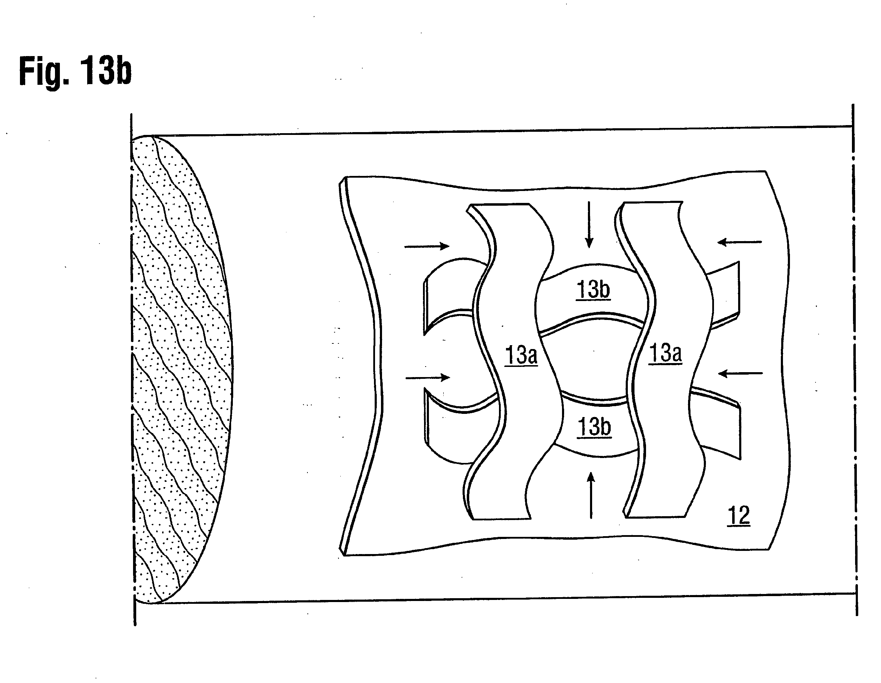

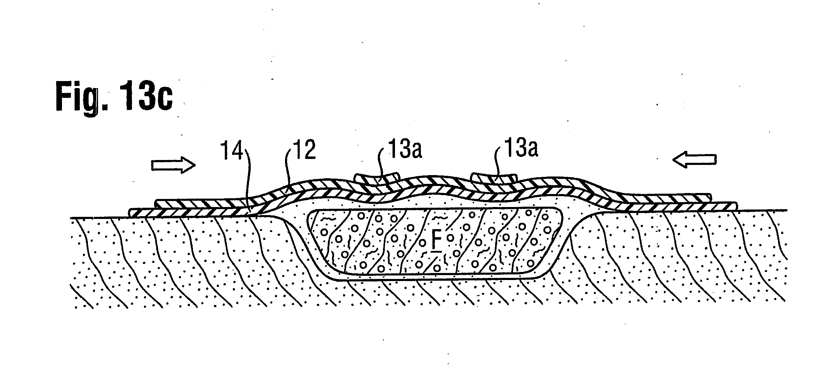

Wie in Figur 13b anhand der Pfeile erkennbar ist, verändern die Streifen 13a, 13b mit der Temperaturveränderung ihre Form und nehmen beispielsweise eine gewellte, verkürzte Form ein. Dadurch wird auch das darunter liegende Grundelement 12 in seiner Form verändert, insbesondere zusammengezogen. Auch hier kommt es zu einer Wundkontraktion und einem steten Zug bzw. Druck auch die Wundränder. Die wirkenden Kräfte F sind auch in Figur 13c nochmals dargestellt.

Wie in dieser Figur erkennbar ist, wird in dieser Ausführungsform die Kavität C vorzugsweise mit einer Wundbettfüllung F, beispielsweise einem Textil, mindestens teilweise ausgefüllt. Eine derartige Wundbettfüllung erübrigt sich in den anderen Ausführungsformen und ist auch hier nur optional. Sie kann jedoch auch in den anderen Ausführungsformen verwendet werden. Auch hier lassen sich wieder die oben genannten Sensoren und Quellen verwenden. Auch eine Unterdruckbeaufschlagung ist möglich.

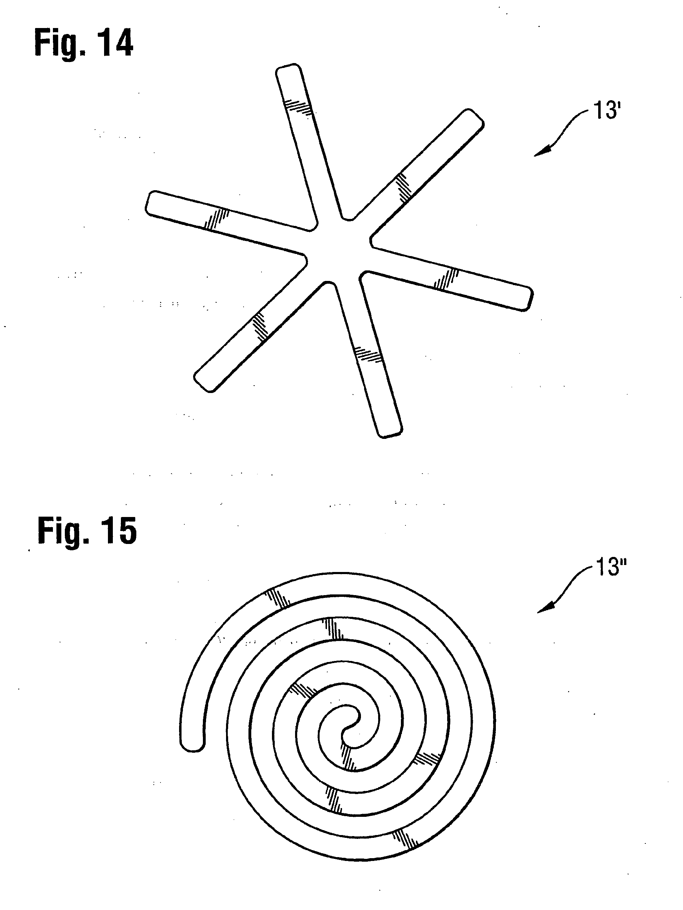

Anstelle der Streifen 13a, 13b lassen sich auch sternförmige Teilelemente 13' (siehe Figur 14), spiralförmige Teilelemente 13" (siehe Figur 15) oder andere Formen aus Formgedächtnis-Materialien verwenden.

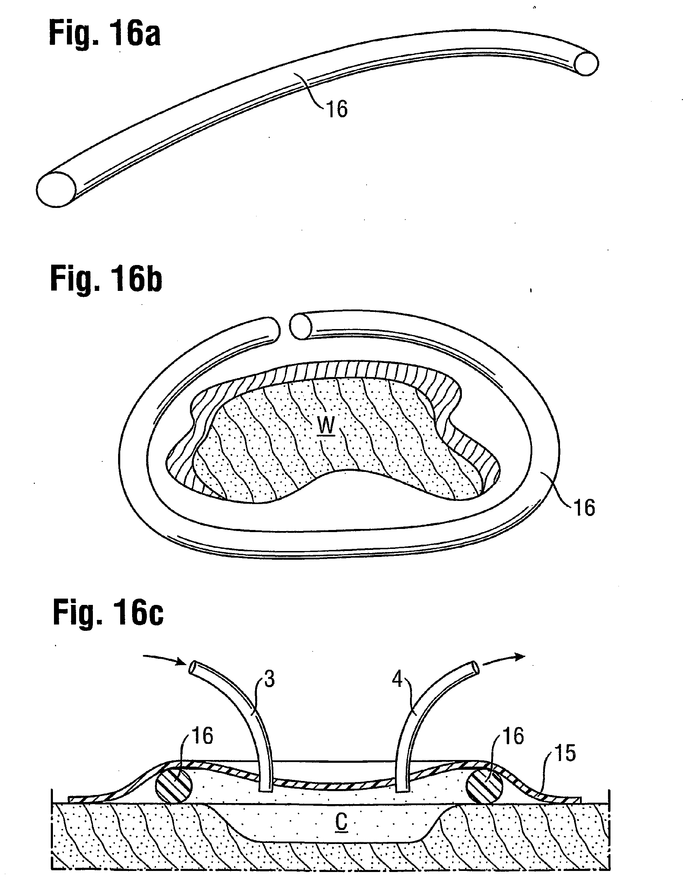

In den Figuren 16a bis 16c ist noch eine weitere Ausführungsform der erfindungsgemässen Vorrichtung dargestellt. Hier ist nun ein biegbares Profilelement oder Strang 16 vorhanden, welches im Normalzustand geradlinig oder leicht gebogen ist, wie dies in Figur 16a erkennbar ist. Es lässt sich ringförmig um die Wunde W legen, wie dies in Figur 16b gezeigt ist. Es wird in seiner Lage mittels einer Abdeck- und Haftfolie 15 gehalten, welche über den durch das Profilelement 16 definierten Innenkreis, über das Profilelement 16 selber und auf die gesunde Haut ausserhalb des Strangs 16 gelegt und dort auf die Haut aufgeklebt wird. Anstelle einer selbsthaftenden Folie kann diese auch an den Rändern mit einem Klebeband angeklebt werden. Vorzugsweise klebt sie jedoch auch auf dem Profilelement 16 fest. Die Folie 15 kann mit Durchgangsöffnungen für die Schläuche 3, 4 versehen sein oder sie kann nun einfach durchgestochen werden, um diese Öffnungen zu erzeugen.

Bei einem Unterdruck in der Kavität C entsteht durch den atmosphärischen Aussendruck eine Kraft auf die Folie 15. Aus der Kraft erfolgt als Resultierende eine Normalkraft auf das ringförmige Profilelement 16 und es kommt zu einer Wundkontraktion. So wird auch ohne Verwendung eines Schwamms im Wundbett verhindert, dass die Folie 15 mit der Wunde verwächst.

Vorzugsweise erhöht sich zudem noch die Profilhöhe des Rings 16 im Gebrauch. Das Profilelement 16 kann dabei entweder ein Formgedächtnis aufweisen, wobei es bei Erreichen der menschlichen Körpertemperatur seinen Durchmesser vergrössert und so die Folie mehr spannt und einen Zug auf die Wundränder ausübt. Es kann jedoch auch einen relativ grossen Wärmeausdehnungskoeffizienten aufweisen. Hierzu wird es vorzugsweise bei einer relativ tiefen Temperatur auf die Wunde aufgesetzt und mit der Folie 15 verbunden, um sich dann bei Erwärmung auszudehnen und die Folie zu spannen.

Die Profilerhöhung kann auch mit mechanischen Mitteln, beispielsweise mit einer im Profilelement eingelagerten Feder oder durch Aufblasen des Profilelements erzielt werden.

Mögliche Materialien für den Strang sind ein Polymer mit Formgedächtnis, beispielsweise Block-Copolymere. Mögliche Materialien für die Folie 15 sind Polymere. Die Folie weist vorzugsweise eine Dicke von 0.1 bis 2 mm auf. Das Profilelement 16 weist vorzugsweise einen Durchmesser von ca. 5 bis 30 mm auf. Vorzugsweise ist das Profilelement ein Vollprofil.

Es versteht sich von selbst, dass die oben genannten Elemente luft- und flüssigkeitsdicht sind, falls mit Unterdruckbeaufschlagung gearbeitet werden soll. Ebenso sind Durchgangsöffnungen entsprechend luft- und flüssigkeitsdicht zu verschliessen.

### Bezugszeichenliste

- 1: Abdeckplatte
- 10: erste Durchgangsöffnung
- 10': zweite Durchgangsöffnung
- 11: Abdeckplattenzuschnitt
- 12: Grundelement
- 13a: Längsstreifen
- 13b: Querstreifen
- 13': sternförmiges Teilelement
- 13": spiralförmiges Teilelement
- 14: Haftfolie
- 15: Abdeck- und Haftfolie
- 16: Profilelement
- 2: Haftfolie
- 3: Zuführschlauch
- 4: Abführschlauch
- 5: Hydrogel
- 6: Ultraschallsender/Photoquelle
- 60: Elektrische Leitung
- 61: Schallwellen/Licht
- 7: Temperatur/Hydrosensor
- 70: Signalleitung
- 8: Durchflusssensor
- 80: Signalleitung
- 9: Heizelement

- A: Gesundes Gewebe
- W: Wunde
- C: Kavität
- V: Vene
- F: Wundbettfüllung

## Patentansprüche

1. Vorrichtung zur Wundbehandlung eines Patienten, wobei die Vorrichtung eine Abdeckung zur Bildung einer Unterdruckkammer über der Wunde aufweist, wobei die Abdeckung auf der die Wunde umgebenden Haut befestigbar ist, **dadurch gekennzeichnet, dass** die Form der Abdeckung nach der Aufbringung auf die Wunde und ihrer Befestigung auf der Haut von einer Basisform in eine vordefinierte Gebrauchsform veränderbar ist, wodurch eine Wundkontraktion stattfindet, und dass die Abdeckung selbsttätig von der Basisform in die Gebrauchsform veränderbar ist.

2. Vorrichtung nach Anspruch 1, wobei die Abdeckung mindestens teilweise, insbesondere vollständig, aus einem Material mit Formgedächtnis besteht.

3. Vorrichtung nach einem der Anspruch 2, wobei die Abdeckung mindestens teilweise aus einem Polymer mit Formgedächtniseigenschaften besteht.

4. Vorrichtung nach einem der Ansprüche 2 bis 3, wobei die Abdeckung bei einer Temperatur, welche annähernd einer Körpertemperatur des Patienten entspricht, die Gebrauchsform aufweist und/oder wobei die Abdeckung bei einer Temperatur, welche annähernd Zimmertemperatur entspricht, die Basisform aufweist.

5. Vorrichtung nach Anspruch 2, wobei die Abdeckung ein Grundelement ohne Formgedächtnis und mindestens ein auf oder in diesem Grundelement angeordnetes Teilelement mit Formgedächtnis aufweist, wobei das mindestens eine Teilelement sich derart über das Grundelement erstreckt, dass es bei Veränderung seiner Form das Grundelement zu einer vorgegebenen Formveränderung zwingt.

6. Vorrichtung nach Anspruch 5, wobei das Grundelement eine annähernd plane Platte ist und das mindestens eine Teilelement ein Streifen ist, welcher sich über die Platte erstreckt.

7. Vorrichtung nach Anspruch 6, wobei mindestens zwei streifenförmige Teilelemente vorhanden sind, welche annähernd rechtwinklig zueinander angeordnet sind oder wobei der Streifen sich spiralförmig über die Platte erstreckt.

8. Vorrichtung nach Anspruch 5, wobei das Grundelement eine annähernd plane Platte ist und das mindestens eine Teilelement sternförmig ausgebildet ist.

9. Vorrichtung nach Anspruch 1, wobei die Abdeckung mit mindestens einem mechanischen Element versehen ist, mittels welchem ihre Form veränderbar ist.

10. Vorrichtung nach Anspruch 9, wobei das mechanische Element mindestens eine Rückstellfeder ist.

11. Vorrichtung nach einem der Ansprüche 1 bis 10, wobei die Abdeckung in der Basisform annähernd plan ausgebildet ist und/oder wobei die Abdeckung in der Gebrauchsform annähernd kalottenförmig ausgebildet ist, wobei die Kalotte sich von der Wunde weg wölbt.

12. Vorrichtung nach einem der Ansprüche 1 bis 11, wobei die Abdeckung mindestens zwei Durchgangsöffnungen zur Durchführung von Zu- bzw. Abführschläuche aufweist.

13. Vorrichtung nach einem der Ansprüche 1 bis 10, wobei die Abdeckung ein zu einem Ring biegbares Profilelement und eine über den Ring spannbare Abdeckfolie aufweist.

14. Vorrichtung nach Anspruch 13, wobei das Profilelement ein Formgedächtnis aufweist.

15. Vorrichtung nach einem der Ansprüche 13 bis 14, wobei die Abdeckfolie aus einem Polymer gefertigt ist.

16. Vorrichtung nach einem der Ansprüche 13 bis 15, wobei die Abdeckfolie mindestens zwei Durchgangslöcher zur Durchführung von Zu- bzw. Abführschläuchen aufweist.

17. Vorrichtung nach einem der Ansprüche 1 bis 16, wobei an oder in der Abdeckung eines oder mehrere der folgenden Mittel angeordnet sind: ein Ultraschallsender, eine Lichtquelle, ein Temperatursensor, ein Sensor zur Messung der Luftfeuchtigkeit, ein Sensor zur Messung der Durchblutung, ein Sensor zur Messung der bakteriellen Besiedelung, eine Heizung.

18. System zur Wundbehandlung mit einer Vorrichtung zur Wundbehandlung gemäss einem der Ansprüche 1 bis 17 und einer damit verbindbaren Vakuumpumpe zur Erzeugung eines Unterdrucks in einer durch die Vorrichtung zur Wundbehandlung erzeugten Kavität.

## Claims

1. A device for treating wounds of a patient, wherein the device comprises a covering for forming a low-pressure chamber over the wound, wherein the covering can be secured on the skin surrounding the wound, **characterized in that**, after the covering has been applied to the wound and secured on the skin, the shape of the covering can change from a basic shape to a predefined shape for use, as a result of which a contraction of the wound takes place, and **in that** the covering can change automatically from the basic shape to the shape for use.

2. The device as claimed in claim 1, wherein the covering is made at least partially, in particular entirely, from a material with shape memory.

3. The device as claimed in claim 2, wherein the covering is made at least partially from a polymer with shape memory properties.

4. The device as claimed in one of claims 2 through 3, wherein the covering has the shape for use at a temperature that corresponds approximately to a body temperature of the patient, and/or wherein the covering has the basic shape at a temperature that corresponds approximately to room temperature.

5. The device as claimed in claim 2, wherein the covering comprises a base element without shape memory and at least one subcomponent with shape memory that is arranged on or in this base element, wherein the at least one subcomponent extends over the base element in such a way that, upon changing shape, it forces the base element to undergo a predetermined change of shape.

6. The device as claimed in claim 5, wherein the base element is an approximately plane plate, and the at least one subcomponent is a strip that extends over the plate.

7. The device as claimed in claim 6, wherein at least two strip-shaped subcomponents are present, which are arranged approximately at right angles to each other, or wherein the strip extends in a spiral shape over the plate.

8. The device as claimed in claim 5, wherein the base element is an approximately plane plate, and the at least one subcomponent is star-shaped.

9. The device as claimed in claim 1, wherein the covering is provided with at least one mechanical element by means of which its shape can be changed.

10. The device as claimed in claim 9, wherein the mechanical element is at least one restoring spring.

11. The device as claimed in one of claims 1 through 10, wherein the covering in the basic shape has an approximately plane configuration, and/or wherein the covering in the shape for use has an approximately dome-shaped configuration in which the dome curves away from the wound.

12. The device as claimed in one of claims 1 through 11, wherein the covering has at least two through-openings for the passage of delivery and drainage tubes.

13. The device as claimed in one of claims 1 through 10, wherein the covering comprises a profile element, which can be bent to form a ring, and a cover film that can be stretched over the ring.

14. The device as claimed in claim 13, wherein the profile element has a shape memory.

15. The device as claimed in one of claims 13 through 14, wherein the cover film is made from a polymer.

16. The device as claimed in one of claims 13 through 15, wherein the cover film has at least two holes for the passage of delivery and drainage tubes.

17. The device as claimed in one of claims 1 through 16, wherein one or more of the following means are arranged on or in the covering: an ultrasound transmitter, a light source, a temperature sensor, a sensor for measuring the air humidity, a sensor for measuring the flow of blood, a sensor for measuring the bacterial colonization, a heating means.

18. A system for treating wounds, with a device for treating wounds as claimed in one of claims 1 through 17 and with a vacuum pump that can be connected to said device and is used to generate a low pressure in a cavity generated by the device for treating wounds.

## Revendications

1. Dispositif de traitement de plaie d'un patient, le dispositif comportant un élément couvrant permettant de former une chambre de sous-pression au-dessus de la plaie, l'élément couvrant pouvant être fixé sur la peau entourant la plaie, **caractérisé en ce que** la forme de l'élément couvrant peut être modifiée, après application sur la plaie et fixation à la peau, en passant d'une forme de base à une forme fonctionnelle prédéfinie permettant de mettre en place une contraction de la plaie et que l'élément couvrant peut être modifié automatiquement de la forme de base dans la forme fonctionnelle.

2. Dispositif selon la revendication 1, l'élément couvrant se composant au moins en partie, notamment entièrement, d'une matière à mémoire de forme.

3. Dispositif selon la revendication 2, l'élément couvrant se composant au moins en partie de polymère présentant des propriétés de mémoire de forme.

4. Dispositif selon l'une quelconque des revendications 2 à 3, l'élément couvrant présentant, à une température proche d'une température du corps du patient, la forme fonctionnelle et/ou l'élément couvrant présentant, à une température correspondant approximativement à la température d'une pièce, la forme de base.

5. Dispositif selon la revendication 2, l'élément couvrant présentant un élément de base sans mémoire de forme et au moins un élément partiel disposé sur ou dans cet élément de base à mémoire de forme, l'au moins un élément partiel s'étendant de telle sorte au-dessus de l'élément de base qu'il contraint, en présence d'une modification de sa forme, l'élément de base dans une forme modifiée prédéfinie.

6. Dispositif selon la revendication 5, l'élément de base prenant approximativement la forme d'une plaque plane et l'au moins un élément partiel prenant la forme d'une bande s'étendant au-dessus de la plaque.

7. Dispositif selon la revendication 6, **caractérisé par** la présence d'au moins deux éléments partiels en forme de bande disposés approximativement perpendiculairement l'un par rapport à l'autre ou la bande s'étendant en spirale au-dessus de la plaque.

8. Dispositif selon la revendication 5, l'élément de base prenant approximativement la forme d'une plaque plane et l'au moins un élément partiel prenant une forme d'étoile.

9. Dispositif selon la revendication 1, l'élément couvrant étant pourvu d'au moins un élément mécanique permettant de faire varier sa forme.

10. Dispositif selon la revendication 9, l'élément mécanique prenant la forme d'au moins un ressort de rappel.

11. Dispositif selon l'une quelconque des revendications 1 à 10, l'élément couvrant étant réalisé de façon au moins approximativement plane dans la forme de base et/ou l'élément couvrant étant réalisé au moins approximativement en forme de calotte dans la forme fonctionnelle, la calotte étant bombée dans un sens d'écartement par rapport à la plaie.

12. Dispositif selon l'une quelconque des revendications 1 à 11, l'élément couvrant comportant au moins deux ouvertures traversantes permettant de faire passer des tuyaux flexibles d'abduction et/ou d'adduction.

13. Dispositif selon l'une quelconque des revendications 1 à 10, l'élément couvrant comportant un élément profilé pouvant être infléchi de façon à former un anneau et un film couvrant pouvant être serré au-dessus de l'anneau.

14. Dispositif selon la revendication 13, l'élément profilé présentant une mémoire de forme.

15. Dispositif selon l'une quelconque des revendications 13 à 14, le film couvrant étant fabriqué en polymère.

16. Dispositif selon l'une quelconque des revendications 13 à 15, le film couvrant comportant au moins deux trous traversants permettant de faire passer des tuyaux flexibles d'abduction et/ou d'adduction.

17. Dispositif selon l'une quelconque des revendications 1 à 16, **caractérisé par** la présence, au niveau ou à l'intérieur de l'élément couvrant, d'un ou de plusieurs des moyens suivants : d'un émetteur à ultrasons, d'une source lumineuse, d'un capteur de température, d'un capteur de mesure de l'humidité de l'air, d'un capteur de mesure de la pression sanguine, d'un capteur de mesure de la colonisation bactérienne, d'un élément de chauffage.

18. Système de traitement de plaie doté d'un dispositif de traitement de plaie selon l'une quelconque des revendications 1 à 17 et d'une pompe à vide pouvant être reliée à l'ensemble pour produire une sous-pression dans une cavité produite par le dispositif de traitement de plaie.
